# EUROPÄISCHE PATENTSCHRIFT

(11) **EP 0 363 753 B1**
(45) Veröffentlichungstag und Bekanntmachung des Hinweises auf die Patenterteilung: **28.06.1995**
(21) Anmeldenummer: 89118051.5
(22) Anmeldetag: 29.09.1989
(51) Int. Cl.: A61F 2/24, B29C 41/14, B29C 41/20, A61L 27/00

(54) **Verfahren zur Herstellung eines Flexiblen Schliessorganes, insbesondere einer Herzklappe**
Process for manufacturing of a flexible closing member, especially a heart valve
Procédé pour fabriquer un organe de fermeture flexible, en particulier une valvule cardiaque

(30) Priorität: 11.10.1988 DE 3834545
(43) Veröffentlichungstag der Anmeldung: 18.04.1990
(73) Patentinhaber: Adiam Medizintechnik GmbH & Co. KG, D-41812 Erkelenz (DE)
(72) Erfinder: Jansen, Josef, Dr.-Ing., D-52064 Aachen (DE); Reul, Helmut, Prof. Dr., D-52353 Düren (DE); Rau, Günther, Prof. Dr., D-52066 Aachen (DE)
(74) Vertreter: Döring, Wolfgang, Dr.-Ing. Patentanwälte Hauck, Graalfs, Wehnert, Döring, Siemons

(56) Entgegenhaltungen:
- EP-A- 0 114 025
- EP-A- 0 193 987
- EP-A- 0 224 153
- GB-A- 2 046 165
- US-A- 4 222 126
- US-A- 4 247 292
- US-A- 4 340 977
- US-A- 4 364 127
- MEDICAL AND BIOLOGICAL ENGINEERING, Band 13, Nr. 4, Juli 1975, Seiten 509-517; W. LENNE et al.: "Total opening valves for a ventricular prosthesis"
- FORSCHUNGSBERICHT, 1983-1984, Seiten 85-89, Helmholtz Institut, RWTH Aachen,DE; M. HEROLD et al.: "Nahtlose Integration von Polyurethan-Taschenklappen-Segeln in verschiedene Klappenringgeometrien"
- BIOMEDIZINISCHE TECHNIK, Band 23, Nr. 7/8, Juli/August 1978, Seiten 173-176; M. WASCHMANN: "Ein neues Verfahren zur Herstellung von integrierten Taschenklappen für künstliche Blutpumpen"

## Beschreibung

Die vorliegende Erfindung betrifft ein Verfahren zum Herstellen von flexiblen Schließorganen, insbesondere Herzklappen, die ein Gehäuse und mindestens ein im Gehäuse angeordnetes und damit verbundenes flexibles segelförmiges Schließelement aufweisen, das zwischen einer offenen und einer geschlossenen quasistabilen Endlage hin- und herbewegbar ist, wobei es seine Krümmung ändert, bei dem man das Schließelement formt und gleichzeitig an das Gehäuse anformt oder ein vorgeformtes Schließelement mit dem Gehäuse verbindet, insbesondere an dieses anformt.

Ein Verfahren der vorstehend beschriebenen Art ist bekannt (Nahtlose Integration von Polyurethan-Taschenklappen-Segeln in verschiedene Klappenringgeometrien von M. Herold, B. Reck aus Forschungsbericht 1983-84, Helmholtz Institut, RWTH Aachen, S. 85-89). Das in dieser Veröffentlichung beschriebene Verfahren dient zur Herstellung einer dreisegligen Herzklappe, wobei man ein vorgefertigtes Klappengehäuse auf einer Tauchform befestigt, welche Formflächen für die zu formenden Schließelemente, d.h. Segel, der Herzklappe aufweist. Diese Formflächen weisen eine zweidimensionale Krümmung auf und geben die Form der Schließelemente im geschlossenen Zustand der Herzklappe wieder. Die Tauchform wird dann mit dem aufgeschobenen Klappengehäuse in eine Polyurethanlösung eingetaucht, wodurch sich auf den Formflächen dünne Lagen aus Polyurethan ausbilden, die die Schließelemente der Klappe bilden. Diese Schließelemente werden gleichzeitig an das Klappengehäuse angeformt. Die Tauchform wird dann aus dem entsprechenden Tauchbad entfernt. Je nach der gewünschten Dicke der Schließelemente kann der Tauchvorgang mehrere Male wiederholt werden. Schließlich werden die trockenen, aber noch miteinander verbundenen Segel getrennt. Anschließend kann die Klappe durch einfaches Abziehen von der Tauchform entformt werden.

Wie vorstehend erwähnt, werden bei diesem bekannten Verfahren die Schließelemente im "geschlossenen Zustand" der Klappe hergestellt, d.h. die Formflächen sind so gekrümmt ausgebildet, daß die sich auf den Formflächen ausbildende flexible Polyurethanlage eine Form besitzt, die die einzelnen Schließelemente im geschlossenen Zustand der Klappe einnehmen. Bei dieser Form handelt es sich in der Regel um eine Teilkugelform, jedenfalls um ein zweifach gekrümmtes Gebilde. Eine derartige Form mit zweidimensionaler Krümmung hat man insbesondere aus Gründen einer besseren Spannungsverteilung über das Schließelement im geschlossenen Zustand desselben gewählt. Bekanntlich stellt hierfür die Kugel ein besonders geeignetes Gebilde dar.

Eine solche vorgeprägte Kugelform der Schließelemente hat jedoch den Nachteil, daß relativ hohe Beuldrücke erforderlich sind, um die nach innen gewölbte Kugelform beim Öffnen der Klappe in eine nach außen gewölbte Kugelform zu überführen. Bei dieser Segelbewegung ist ferner die Gefahr groß, daß Faltungen des Materials innerhalb des Schließelementes auftreten. Diese Faltungen bewirken lokal sehr hohe Biege-Wechsel-Beanspruchungen, die zum frühzeitigen Verschleiß führen. Zudem sind diese Faltungen und die sich daraus ergebende ungenügende Strömungsführung Ursache für das Auslösen von Thrombosierungs- und Hämolysereaktionen aufgrund der auftretenden erhöhten Schubspannungen. Es wird ferner angenommen, daß auch die Neigung zur Kalzifizierung hierdurch erhöht wird.

Wie erwähnt, sind bei den durch das eingangs beschriebene Verfahren hergestellten Herzklappen, die im geschlossenen Zustand hergestellt werden und bei denen entsprechende Vorkrümmungen in die Schließelemente eingebaut werden, sehr hohe, dreidimensionale Verspannkräfte (Beuldrücke) erforderlich, um die Schließelemente von ihrer zur Strömung hin gesehenen konvexen Form (geschlossener Zustand) in die konkave Form (geöffneter Zustand) zu überführen. Der Erfindung liegt die Aufgabe zugrunde, ein Verfahren der angegebenen Art zu schaffen, mit dem ein Schließorgan hergestellt werden kann, das sich durch ein besonders gutes Öffnungs- und Schließverhalten ohne störende Faltenbildung auszeichnet und bei dem besonders niedrige Drücke ausreichen, um die Schließelemente vom geschlossenen in den geöffneten Zustand und umgekehrt zu überführen.

Erfindungsgemäß soll ferner ein Verfahren zur Verfügung gestellt werden, mit dem ein Schließorgan hergestellt werden kann, daß der menschlichen Aortenklappe besonders ähnlich ist und das sich somit in besonders günstiger Weise zur Implantation im menschlichen Herzen eignet. Ein solches Schließorgan soll eine besonders gute Hämodynamik und Dauerfestigkeit aufweisen.

Die vorstehend genannte Aufgabe wird erfindungsgemäß bei dem eingangs beschriebenen Verfahren dadurch gelöst, daß man das Gehäuse radial aufweitet, das Schließelement als im wesentlichen ebenes Flächenelement formt und im aufgeweiteten Zustand des Gehäuses gleichzeitig an dieses anformt oder mit dem aufgeweiteten Gehäuse verbindet, insbesondere an dieses anformt, wobei man das Gehäuse derart aufweitet, daß die sich hierdurch ergebende, gegenüber dem nicht aufgeweiteten Zustand vergrößerte Oberfläche des Schließelementes im Normalzustand des Gehäuses die Einnahme der beiden quasistabilen Endlagen durch das Schließelement ermöglicht, und daß man anschließend das Gehäuse in den Normalzustand bringt.

Bei dem erfindungsgemäßen Verfahren wird somit im Gegensatz zum Stand der Technik das Schließelement nicht im geschlossenen Zustand geformt, sondern in einem zwischen dem geschlossenen und offenen Zustand befindlichen mittleren Zustand, wobei das Schließelement als im wesentlichen ebenes Flächenelement ausgebildet wird. Um zu erreichen, daß dieses ebene Flächenelement sowohl die offene als auch die geschlossene Stellung einnehmen kann, wird es im radial aufgeweiteten Zustand des Gehäuses des Schließorganes geformt und an das Gehäuse angeformt bzw. für den Fall, daß es getrennt vom Gehäuse hergestellt wird, so geformt, daß es an die aufgeweitete Gehäuseform angepaßt ist, wonach es dann mit dem aufgeweiteten Gehäuse verbunden bzw. an dieses angeformt wird. Auf diese Weise erhält das Schließelement eine vergrößerte Oberfläche, die im Normalzustand des Gehäuses zu einer Wölbung des Schließelementes radial nach innen oder außen führt. Diese sich durch die spezielle Art der Formung ergebende überschüssige Oberfläche des Schließelementes wird dabei so bemessen, daß das Schließelement im Normalzustand des Gehäuses die beiden quasistabilen Endlagen im offenen und geschlossenen Zustand einnehmen kann.

Eine Alternative zu dem vorstehend genannten Verfahren ist im Patentanspruch 2 wiedergegeben.

Erfindungsgemäß ist man bestrebt, das Schließelement als im wesentlichen ebenes Flächenelement zu formen, wobei jedoch auch ein in Strömungsrichtung geringfügig einfach gekrümmtes Flächenelement zweckmäßig ist. Diese Ausbildung des Schließelementes wird durch die Formung in einem quasi-mittleren Zustand zwischen dem offenen und geschlossenen Zustand erreicht. Wie erwähnt, wird das Maß für die Aufweitung bzw. Aufspreizung des Gehäuses und damit der Vergrößerung der Oberfläche des Schließelementes durch die beiden Endlagen desselben bestimmt.

Durch das vorstehend beschriebene Herstellverfahren wird erreicht, daß das Schließelement ohne Aufbringung von großen Beuldrücken aus seiner geschlossenen in seine offene Stellung unter Änderung seiner Krümmung überführbar ist. Der Übergang zwischen beiden Endstellungen geschieht dabei faltenlos mit minimalen Bollspannungen durch eine quasi zweidimensionale Rollbewegung. In der offenen Stellung nutzt das Schließelement zudem die begleitenden Strömungskräfte für einen günstigen Schließvorgang und reduziert dadurch die Schließvolumina. Die Segelkinematik entspricht so in hervorragender Weise der von natürlichen Herzklappen.

Durch das erfindungsgemäße Verfahren können die bei bisher üblichen Segelgeometrien aufgeprägten Form-Memory-Effekte, die entschiedend zum Druckverlust und zur Membranfaltenbildung beitragen, ausgeschaltet werden. Dadurch werden zum einen die Dauerfestigkeit erhöht und zum anderen die Energieverluste reduziert. Der Durchgang durch diese Mittellage (den Herstellzustand) bei jeder Herzaktion ist sowohl für den Öffnungs- als auch für den Schließvorgang energetisch und strömungstechnisch günstig bei besonders geringer Belastung des Segelmaterials.

Bei einer zweckmäßigen Ausgestaltung des erfindungsgemäßen Verfahrens macht man sich das bekannte Tauchverfahren zunutze. Hierbei formt man das Schließelement im möglichst ebenen Zustand durch ein Tauchverfahren mit mindestens einem Tauchvorgang, wobei hierdurch gleichzeitig das Schließelement an das dann radial aufgeweitete Gehäuse angeformt wird.

In Weiterbildung des erfindungsgemäßen Verfahrens weitet man das Gehäuse in Strömungsrichtung auf, wobei man vorzugsweise die Aufweitung unter einem Öffnungswinkel von 2-20° vornimmt.

Zur Durchführung der Aufweitung des Gehäuses macht man sich die bereits bei dem bekannten Tauchverfahren verwendete Tauchform in der Form eines Dornes zunutze. Wie erwähnt, wurde bei dem bekannten Verfahren das Gehäuse auf den Dorn aufgeschoben und daran arretiert, wonach der Formvorgang des Schließelementes durchgeführt wurde. Erfindungsgemäß wird nunmehr ein Dorn eingesetzt, der eine radiale Aufweitung des Gehäuses, insbesondere eine konische Aufweitung desselben in Strömungsrichtung, bewirkt. Desweiteren unterscheidet sich dieser Dorn von dem bei dem bekannten Verfahren eingesetzten Dorn dadurch, daß der Dorn eine im wesentlichen ebene Formfläche, ggf. eine in Strömungsrichtung geringfügig gekrümmte Formfläche, aufweist.

Bei einer speziellen Ausführungsform des erfindungsgemäßen Verfahrens stellt man ein drei Schließelemente aufweisendes Schließorgan (Herzklappe) durch Aufweiten eines drei Pfosten besitzenden Gehäuses und Anformen bzw. Verbinden der Schließelemente (Segel) an das bzw. mit dem Gehäuse her. Hierbei wird das Gehäuse auf die sich in Strömungsrichtung konisch erweiternde Tauchform (Dorn) aufgeschoben, wobei die Pfosten des Gehäuses radial aufgespreizt werden. Entsprechend der gewünschten Endschichtdicke der Segel wird der so vorbereitete Dorn ein- oder mehrmals in eine entsprechende Kunststofflösung, insbesondere Polyurethan, eingetaucht, herausgezogen und anschließend getrocknet. Nach dem eigentlichen Formgebungsprozeß der drei Schließelemente wird die hergestellte Klappe entsprechend dem Linienzug der freien Segelenden mittels eines geeigneten Verfahrens geschnitten und vom Dorn entfernt. Das Gehäuse dieser so gefertigten Herzklappe verformt sich anschließend mit den entsprechenden Segeln in deren offenen (systolischen) Zustand. In diesem Zustand besitzt das Gehäuse eine kreiszylindrische Form, an die sich das Schließelement anpaßt, d.h. den Teil einer Zylindermantelfläche bildet.

Als bevorzugtes Herstellungsmaterial für die Schließelemente kommen geeignete blut- und gewebeverträgliche Kunststoffe, insbesondere Polyurethan, zur Anwendung.

Das auf diese Weise mit dem Schließelement versehene Gehäuse kann anschließend mit einem geeigneten Nahtring versehen werden, der eine Befestigung am entsprechenden Gewebe ermöglicht.

Bei einer speziellen Variante des erfindungsgemäßen Verfahrens, bei der man einen möglichst glatten Übergang zwischen Gehäuse und Schließelement erzielen will, geht man so vor, daß man den Dorn bzw. die Tauchform zuerst durch Tauchen mit mindestens einer ersten Materialschicht überzieht, dann das Gehäuse auf die Tauchform aufschiebt und entsprechend aufweitet und schließlich durch Tauchen mindestens eine zweite Materialschicht aufbringt. Man kann dann die entsprechende Schicht umstülpen und in einer Außenrille mit dem Gehäuse verkleben bzw. verschweißen. Der darüber gesetzte Nahtring verdeckt die entsprechende Verbindungsstelle.

Es versteht sich, daß das vorstehend beschriebene erfindungsgemäße Verfahren nicht allein zur Herstellung von Herzklappen geeignet ist. Es können hiermit vielmehr auch andere Schließorgane (Ventile), beispielsweise für Blutpumpen, hergestellt werden. Wie erwähnt, werden für die Herstellung vorzugsweise Kunststoffe eingesetzt; es ist jedoch auch eine Herstellung mit durch Züchtung gewonnenem biologischen Gewebe möglich. Auch die Zahl der Schließelemente kann variabel sein; so ist das Verfahren insbesondere zur Herstellung von Schließorganen mit einem, zwei und drei Schließelementen geeignet. Dies schließt jedoch nicht aus, daß auch Organe mit einer darüber hinausgehenden Anzahl von Schließelementen hergestellt werden können.

Die erfindungsgemäß hergestellten Schließorgane besitzen den weiteren Vorteil, daß sie sehr gut schließen und sehr weit öffnen, d.h. im geöffneten Zustand allenfalls eine geringe Strömungsbeeinflussung bewirken. So bewirkt beispielsweise bei einer Klappe mit zylindrischem Gehäuse die Strömung eine Öffnung des Schließelementes nahezu in die vollständige Zylinderstellung, so daß sich im Bereich des geöffneten Schließelementes laminare Strömungsverhältnisse ohne Turbulenzen ausbilden. Durch die vorgegebene zweidimensionale Krümmung, die durch das eingangs beschriebene Verfahren nach dem Stand der Technik hergestellt wird, können diese Verhältnisse nicht erreicht werden.

Als bevorzugte Materialstärke für das Schließelement gelangt ein Bereich von 50 - 800 »m, insbesondere 100 - 300 »m, zur Anwendung, wobei bei biologischem Material 600 -800 »m bevorzugt werden.

Das vorstehend beschriebene erfindungsgemäße Verfahren läßt sich neben dem erwähnten Tauchverfahren auch durch andere Kunststoffverarbeitungsverfahren verwirklichen, beispielsweise durch Pressen, Spritzpressen, Spritzgießen, Thermoformen und elektrostatisches Beflocken. Das erwähnte Tauchverfahren wird jedoch bevorzugt verwendet, da es die Formung des Schließelementes und dessen Anformung an das Gehäuse in einem Arbeitsschritt ermöglicht. Bei getrennter Herstellung des Schließelementes kann die Verbindung desselben mit dem Gehäuse durch ein thermisches Verfahren oder ein Klebeverfahren erfolgen. Das getrennt hergestellte Schließelement besitzt hierbei beispielsweise die Form einer ebenen Folie.

Die Schließelemente (Segel) können auch integriert mit sogenannten Bulben (Sinus valsalvae) ähnlich der natürlichen Aortenklappen gefertigt werden. Dabei kann eine solche Bulbenklappe in künstliche Blutpumpen oder als Conduit-Klappenimplantat zum Ersatz geschädigter Aortenklappen eingesetzt werden. Der Conduit kann dabei nur die Form eines zylindrischen Schlauches annehmen. Die Segel können auch direkt in eine künstliche Blutpumpe ohne Bulben integriert werden.

Die Erfindung wird nachfolgend anhand von Ausführungsbeispielen in Verbindung mit der Zeichnung im einzelnen erläutert. Es zeigen:
- Figur 1: eine zur Praktizierung des erfindungsgemäßen Verfahrens verwendete Tauchform, auf der sich ein Gehäuse im aufgeweiten Zustand befindet;
- Figur 2: eine nach dem erfindungsgemäßen Verfahren ausgebildete Dreisegel-Herzklappe im geschlossenen Zustand;
- Figur 3: die Herzklappe der Figur 2 im geöffneten Zustand;
- Figur 4: eine Draufsicht auf die Herzklappe im geschlossenen Zustand; und
- Figur 5: eine Draufsicht auf die Herzklappe im geöffneten Zustand.

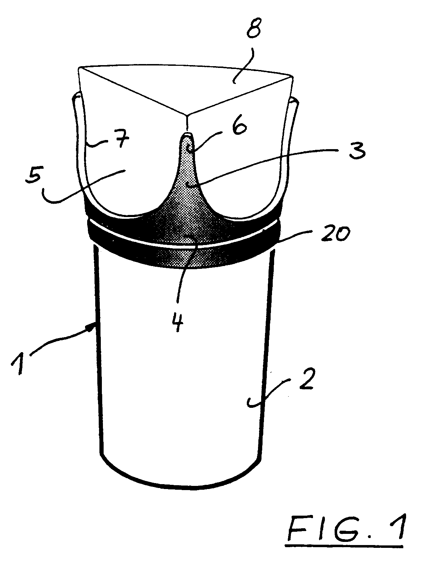

Die in Figur 1 dargestellte Tauchform (Dorn) 1 besteht aus einem zylindrischen Abschnitt 2, einem konischen Abschnitt 3, der in der Draufsicht dreiecksförmig ausgebildet ist, wie bei 8 gezeigt. Die Tauchform besteht vorzugsweise aus Edelstahl, auf dem die auszubildenden Schließelemente geformt werden können. Im Bereich des konischen Abschnitts 3 befinden sich drei Formflächen 5, die im wesentlichen eben sind (zum oberen Ende der Tauchform hin leicht gekrümmt sind).

Zur Herstellung einer künstlichen Dreisegel-Herzklappe wird ein vorgefertigtes, im wesentlichen zylindrisches Klappengehäuse 4, das drei Taschen zur Aufnahme der entsprechenden Schließelemente aufweist, vom zylindrischen Ende der Tauchform her auf diese aufgeschoben. Das Gehäuse wird dabei soweit aufgeschoben, bis es auf den konischen Abschnitt 3 zu sitzen kommt und dabei radial aufgeweitet wird. Die Grenze zwischen dem konischen Abschnitt 3 und dem zylindrischen Abschnitt 2 liegt dabei etwa am tiefsten Punkt der Gehäusetaschen.

Das Gehäuse wird dann so justiert, daß seine Pfosten 6 auf den entsprechenden Lagerflächen (zwischen den Formflächen) des konischen Abschnitts zu liegen kommen. In diesem Zustand wird die Tauchform ein oder mehrere Male in ein entsprechendes Bad einer Kunststofflösung eingetaucht, wobei sich auf den Formflächen 5 eine flexible Kunststoffolie ausbildet, die sich an den Verbindungslinien 7 an das Gehäuse anformt. Nach Beendigung des Tauchvorganges und einem entsprechenden Trocknungsvorgang werden die auf den Formflächen 5 ausgebildeten Schließelemente zurecht geschnitten, wonach die fertige Herzklappe von der Tauchform entfernt wird. Die Klappe kann dann mit einem entsprechenden Nahtring versehen werden, der in der hierzu vorgesehenen Nut 20 befestigt wird.

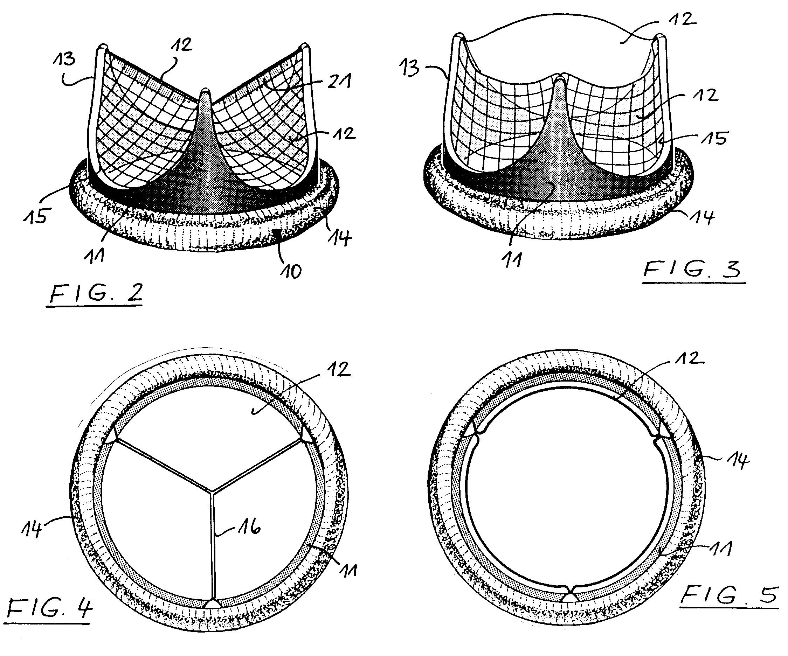

Eine Dreisegel-Herzklappe, die vorzugsweise nach dem in Verbindung mit Figur 1 beschriebenen Verfahren hergestellt worden ist, ist in den Figuren 2 bis 4 dargestellt. Figur 2 zeigt die aus einem Nahtring 14, dreisegelförmigen flexiblen Schließelementen 12 und einem Gehäuse 11 bestehende Herzklappe 10 im geschlossenen Zustand der Schließelemente, während Figur 3 die Herzklappe im offenen Zustand der Schließelemente zeigt. Das Gehäuse 11 der Herzklappe entspricht im wesentlichen dem in Verbindung mit Figur 1 beschriebenen Gehäuse. Die drei Schließelemente sind an den Verbindungslinien 15 an das Gehäuse angeformt. Wie man bei den in Figur 2 dargestellten Schließelementen erkennen kann, besitzen diese im geschlossenen Zustand im wesentlichen die Form eines Teiles der Mantelfläche einer zur Achse des Schließorganes geneigten Kreiszylinders, wobei sich diese Teile im druckbelasteten Zustand zur Mitte des Schließorganes hin kontinuierlich in eine verjüngende Rotationsform wandelt. In diesem geschlossenen Zustand legen sich die drei Schließelemente mit ihren Oberkanten aneinander (wie bei 21 gezeigt), so daß auf diese Weise der entsprechende Durchflußquerschnitt verschlossen wird.

Wie man Figur 3 entnehmen kann, besitzen im geöffneten Zustand der Schließelemente 12 diese im wesentlichen die Form einer Zylindermantelteilfläche, d.h. die Schließelemente setzen die durch die Pfosten 13 des Gehäuses vorgegebene Zylinderkrümmung fort. Die Schließelemente bilden daher in diesem Zustand nahezu eine Fortsetzung des Gehäuses, durch die die drei Gehäusetaschen ausgefüllt werden.

Figur 4 zeigt die Herzklappe der Figuren 2 und 3 im geschlossenen Zustand der Schließelemente. Diese legen sich im geschlossenen Zustand mit ihren Oberkanten aneinander an (bei 21), wobei die Oberkante eines Schließelemente dabei die Form der beiden Schenkel eines Dreiecks einnimmt. Figur 5 zeigt die Herzklappe im geöffneten Zustand in der Draufsicht.

## Patentansprüche

1. Verfahren zum Herstellen von flexiblen Schließorganen, insbesondere künstlichen Herzklappen, die ein Gehäuse und mindestens ein im Gehäuse angeordnetes und damit verbundenes flexibles segelförmiges Schließelement aufweisen, das zwischen einer offenen und einer geschlossenen quasistabilen Endlage hin- und herbewegbar ist, wobei es seine Krümmung ändert, bei dem man das Schließelement formt und gleichzeitig an das Gehäuse anformt oder ein vorgeformtes Schließelement mit dem Gehäuse verbindet, insbesondere an dieses anformt, **dadurch gekennzeichnet**, daß man das Gehäuse radial aufweitet, das Schließelement als im wesentlichen ebenes Flächenelement formt und im aufgeweiteten Zustand des Gehäuses gleichzeitig an dieses anformt oder mit dem aufgeweiteten Gehäuse verbindet, insbesondere an dieses anformt, wobei man das Gehäuse derart aufweitet, daß die sich hierdurch ergebende, gegenüber dem nicht aufgeweiteten Zustand vergrößerte Oberfläche des Schließelementes im Normalzustand des Gehäuses die Einnahme der beiden quasistabilen Endlagen durch das Schließelement ermöglicht, und daß man anschließend das Gehäuse in den Normalzustand bringt.

2. Verfahren zum Herstellen von flexiblen Schließorganen, insbesondere künstlichen Herzklappen, die ein Gehäuse und mindestens ein im Gehäuse angeordnetes und damit verbundenes flexibles segelförmiges Schließorgan aufweisen, das zwischen einer offenen und einer geschlossenen quasistabilen Endlage hin- und herbewegbar ist, wobei es seine Krümmung ändert, bei dem man das Schließelement formt und gleichzeitig an das Gehäuse anformt oder ein vorgeformtes Schließelement mit dem Gehäuse verbindet, insbesondere an dieses anformt, **dadurch gekennzeichnet**, daß man ein radial erweitertes Gehäuse herstellt, das Schließelement als im wesentlichen ebenes Flächenelement formt und gleichzeitig mit der Herstellung des Gehäuses dieses anformt oder bei getrennter Herstellung mit dem Gehäuase verbindet, insbesondere an dieses anformt, und daß man das Gehäuse durch Aufbringung äußerer Kräfte aus dem Herstellungszustand in einen Zustand verformt, in dem sich eine gegenüber dem nicht erweiterten Zustand vergrößerte Oberfläche des Schließelementes ergibt, die die Einnahme der beiden quasistabilen Endlagen durch das Schließelement ermöglicht.

3. Verfahren nach Anspruch 1 oder 2, **dadurch gekennzeichnet**, daß man das Schließelement entsprechend einer Minimalfläche formt, bei der die mittlere Krümmung an jedem Punkt der Oberfläche gleich Null ist.

4. Verfahren nach einem der vorangehenden Ansprüche, **dadurch gekennzeichnet**, daß man das Schließelement als in Strömungsrichtung geringfügig einfach gekrümmtes Flächenelement formt.

5. Verfahren nach einem der Ansprüche 1, 3 oder 4, **dadurch gekennzeichnet**, daß man das Schließelement durch ein Tauchverfahren mit mindestens einem Tauchvorgang formt und gleichzeitig an das Gehäuse anformt.

6. Verfahren nach einem der Ansprüche 1 oder 3 -5, **dadurch gekennzeichnet**, daß man das Gehäuse in Strömungsrichtung aufweitet, insbesondere konisch aufweitet.

7. Verfahren nach Anspruch 6, **dadurch gekennzeichnet**, daß man mit einem Öffnungswinkel von 2 - 20° aufweitet.

8. Verfahren nach einem der Ansprüche 1 oder 3 bis 7, **dadurch gekennzeichnet**, daß man das Gehäuse mit Hilfe eines Dornes radial aufweitet, der mit einer im wesentlichen ebenen Formfläche für das Schließelement versehen ist.

9. Verfahren nach Anspruch 8, **dadurch gekennzeichnet**, daß man mit einem Dorn aufweitet, der mit einer in Strömungsrichtung geringfügig gekrümmten Formfläche für das Schließelement versehen ist.

10. Verfahren nach Anspruch 8 oder 9, **dadurch gekennzeichnet**, daß man das Gehäuse mit einem sich in Strömungsrichtung erweiternden Dorn, insbesondere konisch erweiternden Dorn, aufweitet.

11. Verfahren nach einem der vorangehenden Ansprüche, **dadurch gekennzeichnet**, daß man das Schließelement aus hochflexiblem biokompatiblen Kunststoff, insbesondere Polyurethan, herstellt.

12. Verfahren nach einem der Ansprüche 8 bis 11, **dadurch gekennzeichnet**, daß man den Dorn durch Tauchen mit mindestens einer ersten Materialschicht überzieht, dann das Gehäuse aufschiebt und aufweitet und dann durch Tauchen mindestens eine zweite Materialschicht auf die Formfläche aufbringt.

13. Verfahren nach einem der vorangehenden Ansprüche, **dadurch gekennzeichnet**, daß man ein Dreischließelemente aufweisendes Schließorgan durch Aufweiten eines Dreipfosten besitzenden Gehäuses und Anformen bzw. Verbinden der Schließelemente an das bzw. mit dem Gehäuse herstellt oder ein solches Schließorgan durch Herstellen eines sich erweiternden Gehäuses und Aufbringung äußerer Kräfte fertigt.

14. Verfahren nach Anspruch 1 oder 2, **dadurch gekennzeichnet**, daß man ein fertig hergestelltes Schließelement nachträglich mit Hilfe eines thermischen Verfahrens oder eines Klebeverfahrens mit dem Gehäuse verbindet.

15. Verfahren nach einem der Ansprüche 1 bis 10 und 12 bis 14, **dadurch gekennzeichnet**, daß man das Schließelement aus einem biologischen Gewebe, beispielsweise Pericardium, Fascia Lata, Duramater oder in vivo bzw. ex vivo gezüchtetem Materialgewebe, herstellt.

16. Verfahren nach einem der vorangehenden Ansprüche, **dadurch gekennzeichnet**, daß man zur Herstellung des Schließelementes eines Formfläche verwendet, bei der das Schließelement abweichend von der mittleren Lage durch geringe Vorkrümmungen in seine geschlossene bzw. offene Position verschoben wird.

17. Verfahren nach Anspruch 16, **dadurch gekennzeichnet**, daß man die Vorkrümmung derart bemißt, daß die mittlere Krümmung H der Fläche des Schließelementes und des freien Endes desselben im Verhältnis zum Klappeninnendurchmesser dᵢ zwischen -0,6< Hdᵢ/2 < 0,6 liegt, wobei der Kurvenzug des freien Schließelementrandes insbesondere zwischen -0,3 und + 0,3 liegt.

18. Verfahren nach einem der vorangehenden Ansprüche, **dadurch gekennzeichnet**, daß das Schließelement integriert mit Bulben ähnlich der natürlichen Aortenklappe gefertigt wird.

19. Verfahren nach Anspruch 18, **dadurch gekennzeichnet**, daß das Schließorgan als Conduit-Klappenimplantat gefertigt wird, wobei der Conduit mit Bulben versehen ist oder nur die Form eines zylindrischen Schlauches annimmt.

20. Verfahren nach einem der Ansprüche 1 bis 17, **dadurch gekennzeichnet**, daß das Schließelement direkt in eine künstliche Blutpumpe ohne Bulben integriert wird.

21. Verfahren nach einem der vorangehenden Ansprüche, **dadurch gekennzeichnet**, daß es zum Herstellen von ein-, zwei- und mehrflügeligen Herzklappen eingesetzt wird.

## Claims

1. A method of producing flexible closing members, especially artificial heart valves, said closing members having a housing and at least one flexible sail-like closing element disposed within said housing and connected therewith, said closing element being movable between an open and a closed quasi-stable end position, wherein it changes its curvature, according to which the closing element is formed and is simultaneously formed to said housing or a pre-formed closing element is joined to said housing, especially formed to said housing, characterized by the following steps:
radially expanding said housing,
forming the closing element as a substantially two-dimensional element and simultaneously forming the same to the housing in the expanded condition thereof or joining the closing element to the expanded housing, especially forming to the same, wherein the housing is expanded in such a manner that the surface of the closing element resulting hereby and enlarged with regard to the non-expanded condition enables the occupation of the two quasi-stable end positions by the closing element, and
subsequently bringing said housing into the normal condition.

2. A method of producing flexible closing members, especially artificial heart valves, said closing members comprising a housing and at least one flexible sail-like closing element disposed in said housing and connected therewith, said closing element being movable between an open and a closed quasi-stable end position, wherein it changes its curvature, according to which the closing element is formed and simultaneously formed to said housing or a pre-formed closing element is joined to said housing, especially formed to said housing, characterized by the following steps:
producing a radially expanded housing,
forming the closing element as a substantially two-dimensional element and simultaneously with the production of the housing forming the same to the housing, or with separate production, joining the same to the housing, especially forming to the housing, and deforming said housing by the application of outer forces from the condition of production into a condition in which a surface of said closing element results which is enlarged with regard to the non-expanded condition, the enlarged surface enabling the occupation of the two quasi-stable end positions by the closing element.

3. The method according to claim 1 or 2, characterized by forming said closing element in accordance with a minimum surface according to which the mean curvature at each point of the surface is zero.

4. The method according to one of the preceding claims, characterized by forming said closing element as, in flow direction, slightly simply curved two-dimensional element.

5. The method according to one of the claims 1, 3 or 4, characterized by forming said closing element by a dip method with at least one dip step and simultaneously forming the same to said housing.

6. The method according to one of the claims 1 or 3 - 5, characterized by expanding said housing in flow direction, especially conically expanding.

7. The method according to claim 6, characterized by expanding with an apex angle of 2 - 20°.

8. The method according to one of the claims 1 or 3 to 7, characterized by radially expanding said housing by means of a mandrel which is provided with a substantially plane shaping surface for said closing element.

9. The method according to claim 8, characterized by expanding with a mandrel which is provided with a shaping surface for said closing element slightly curved in flow direction.

10. The method according to claim 8 or 9, characterized by expanding said housing with a mandrel expanding in flow direction, especially conically expanding.

11. The method according to one of the preceding claims, characterized by producing said closing element from highly flexible biocompatible plastics, especially polyurethane.

12. The method according to on of the claims 8 to 11, characterized by coating said mandrel by dipping with at least one first material layer, subsequently slipping-on said housing and expanding and then applying at least one second material layer onto the shaping surface by dipping.

13. The method according to one of the preceding claims, characterized by producing a closing member having three closing elements by expanding a housing having three posts and by forming or connecting the closing elements to said housing or producing such a closing member by manufacturing an expanding housing and by the application of outer forces.

14. The method according to claim 1 or 2, characterized by subsequently joining a completely produced closing element to said housing by means of a thermal process or a bonding process.

15. The method according to one of the claims 1 to 10 and 12 to 14, characterized by producing the closing element from a biological tissue, for instance pericardium, fascia lata, duramater or from in vivo or ex vivo cultivated material tissue.

16. The method according to one of the preceding claims, characterized by using a shaping surface for the production of the closing element according to which the closing element, deviating from the central position, is displaced into its closed or open position by slight precurvatures.

17. The method according to claim 16, characterized by dimensioning the precurvatures such that the mean curvature H of the surface of the closing element and of the free end thereof is in a relation to the inner diameter dᵢ of the valve of between -0,6 < Hd₁/2 < 0,6, wherein the curve train of the free closing element edge is especially between -0,3 and +0,3.

18. The method according to one of the preceding claims, characterized by producing the closing element integrally with bulbs similar to the natural aorta valve.

19. The method according to claim 18, characterized by producing said closing member as conduit-valve-prothesis wherein the conduit is provided with bulbs or has only the shape of a cylindrical hose.

20. The method according to one of the claims 1 to 17, characterized by directly integrating the closing element into an artificial blood pump without bulbs.

21. The method according to one of the preceding claims, characterized by using the same for the manufacture of heart valves having one wing, two wings or a plurality of wings.

## Revendications

1. Procédé de fabrication d'organes de fermeture flexibles, en particulier de valvules cardiaques artificielles, qui présentent un boîtier et au moins un élément de fermeture flexible en forme de voile disposé dans le boîtier et relié à celui-ci, cet élément de fermeture pouvant être déplacé en va-et-vient entre une position extrême ouverte et une position extrême fermée quasi stables tandis que sa courbure se modifie, et dans lequel on forme l'élément de fermeture et on le façonne en même temps sur le boîtier, ou on relie au boîtier, en particulier par façonnage sur celui-ci, un élément de fermeture préalablement formé, caractérisé en ce qu'on élargit le boîtier radialement, qu'on forme l'élément de fermeture comme élément plat essentiellement plan et qu'on le façonne en même temps sur le boîtier lorsque celui-ci est à l'état élargi, ou qu'on le relie au boîtier élargi, en particulier qu'on le façonne sur ce dernier en élargissant le boîtier de telle sorte que la surface ainsi formée de l'élément de fermeture, agrandie par rapport à l'état non élargi, permet à l'élément de fermeture de prendre les deux positions extrêmes quasi stables lorsque le boîtier est dans l'état normal, et en ce qu'ensuite on amène le boîtier dans l'état normal.

2. Procédé de fabrication d'organes de fermeture flexibles, en particulier de valvules cardiaques artificielles, qui présentent un boîtier et au moins un élément de fermeture flexible en forme de voile disposé dans le boîtier et relié à celui-ci, cet élément de fermeture pouvant être déplacé en va-et-vient entre une position extrême ouverte et une position extrême fermée quasi stables tandis que sa courbure se modifie, et dans lequel on forme l'élément de fermeture et on le façonne en même temps sur le boîtier, ou on relie au boîtier, en particulier par façonnage sur celui-ci, un élément de fermeture préalablement formé, caractérisé en ce qu'on fabrique un boitier élargi radialement, qu'on forme l'élément de fermeture comme élément plat essentiellement plan et qu'on le forme en même temps que la fabrication du boîtier ou, dans le cas d'une fabrication séparée, qu'on le relie au boîtier, et en particulier qu'on le forme sur celui-ci, et en ce qu'en exerçant une force externe, on déforme le boîtier depuis son état à la fabrication jusque dans un état dans lequel on obtient une surface de l'élément de fermeture qui est agrandie par rapport à l'état non élargi, et qui permet à l'élément de fermeture de prendre les deux positions extrêmes quasi stables.

3. Procédé selon la revendication 1 ou 2, caractérisé en ce que l'on forme l'élément de fermeture en fonction d'une surface minimale pour laquelle la courbure moyenne est égale à zéro en chaque point de la surface.

4. Procédé selon l'une quelconque des revendications précédentes, caractérisé en ce que l'on forme l'élément de fermeture comme élément plat présentant une légère courbure simple dans la direction de l'écoulement.

5. Procédé selon l'une quelconque des revendications 1, 3 ou 4, caractérisé en ce que l'on forme l'élément de fermeture par un procédé par immersion, avec au moins une opération d'immersion, et qu'on le façonne en même temps sur le boîtier.

6. Procédé selon l'une quelconque des revendications 1 ou 3-5, caractérisé en ce que l'on élargit le boîtier dans la direction de l'écoulement, et qu'en particulier on l'élargit coniquement.

7. Procédé selon la revendication 6, caractérisé en ce que l'on réalise l'élargissement avec un angle d'ouverture de 2-20°.

8. Procédé selon l'une quelconque des revendications 1 ou 3 à 7, caractérisé en ce que l'on élargit radialement le boîtier à l'aide d'un mandrin qui est doté d'une surface essentiellement plane pour le formage de l'élément de fermeture.

9. Procédé selon la revendication 8, caractérisé en ce que l'on effectue l'élargissement avec un mandrin qui est doté d'une surface légèrement incurvée dans la direction de l'écoulement pour le formage de l'élément de fermeture.

10. Procédé selon la revendication 8 ou 9, caractérisé en ce que l'on élargit le boîtier avec un mandrin s'élargissant dans la direction de l'écoulement, et en particulier avec un mandrin qui s'élargit coniquement.

11. Procédé selon l'une des revendications précédentes, caractérisé en ce que l'on réalise l'élément de fermeture en plastique biocompatible très flexible, et en particulier en polyuréthane.

12. Procédé selon l'une quelconque des revendications 8 à 11, caractérisé en ce que l'on recouvre le mandrin d'au moins une première couche de matière, par immersion, qu'on déploie alors le boîtier et qu'on l'élargit, et qu'on applique alors au moins une seconde couche de matière sur la surface de formage, par immersion.

13. Procédé selon l'une quelconque des revendications précédentes, caractérisé en ce que l'on fabrique un organe de fermeture présentant un élément triple de fermeture, par élargissement d'un boîtier possédant un tripode et façonnage des éléments de fermeture sur le boîtier ou liaison des éléments de fermeture à celui-ci, ou que l'on fabrique un tel organe de fermeture par fabrication d'un boîtier s'élargissant et application de forces externes.

14. Procédé selon la revendication 1 ou 2, caractérisé en ce qu'à l'aide d'un procédé thermique ou d'un procédé de collage, on relie ultérieurement au boîtier un élément de fermeture préalablement fabriqué.

15. Procédé selon l'une quelconque des revendications 1 à 10 et 12 à 14, caractérisé en ce que l'on fabrique l'élément de fermeture à partir d'un tissu biologique, par exemple de péricarde, la Fascia Lata, la dure-mère, ou d'un tissu de matière cultivée in vivo ou ex vivo.

16. Procédé selon l'une quelconque des revendications précédentes, caractérisé en ce que, pour la fabrication de l'élément de fermeture, on utilise une surface de formage telle qu'en partant de sa position médiane, l'élément de fermeture est déplacé dans sa position fermée ou sa position ouverte par de petites courbures préalables.

17. Procédé selon la revendication 16, caractérisé en ce que l'on dimensionne la courbure préalable de telle sorte que le rapport entre la courbure médiane H de la surface de l'élément de fermeture et de l'extrémité libre de celui-ci et le diamètre interne d₁ de la valvule soit tel que -0,6 < Hd₁/2 < 0,6, tandis que la courbure du bord libre de l'élément de fermeture vaut en particulier entre -0,3 et +0,3.

18. Procédé selon l'une quelconque des revendications précédentes, caractérisé en ce que l'élément de fermeture est fabriqué avec des bulbes intégrés, de manière similaire à la valvule aortique naturelle.

19. Procédé selon la revendication 18, caractérisé en ce que l'organe de fermeture est fabriqué comme implant de valvule de conduit, le conduit étant doté de bulbes ou prenant uniquement la forme d'un tube cylindrique flexible.

20. Procédé selon l'une quelconque des revendications 1 à 17, caractérisé en ce que l'élément de fermeture est directement intégré dans une pompe sanguine artificielle sans bulbes.

21. Procédé selon l'une quelconque des revendications précédentes, caractérisé en ce qu'il est utilisé pour la fabrication de valvules cardiaques à un, deux et plusieurs lobes.
